(19) Europäisches Patentamt
European Patent Office
Office européen des brevets

(11) Veröffentlichungsnummer: **0 061 657**
**B1**

(12) # EUROPÄISCHE PATENTSCHRIFT

(45) Veröffentlichungstag der Patentschrift: **20.06.84**

(51) Int. Cl.³: **C 07 C 102/00**, C 07 C 103/127, C 07 C 103/34

(21) Anmeldenummer: **82102187.0**

(22) Anmeldetag: **17.03.82**

(54) **Verfahren zur Herstellung von 2-Chloracetessigsäureamiden.**

(30) Priorität: **26.03.81 CH 2044/81**
**17.09.81 CH 6004/81**

(43) Veröffentlichungstag der Anmeldung: **06.10.82 Patentblatt 82/40**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen:
**EP - A - 0 005 849**
**EP - A - 0 037 015**
**DE - A - 1 931 964**
**US - A - 3 852 351**
**US - A - 4 093 654**
**US - A - 4 214 002**

**JOURNAL OF THE AMERICAN CHEMICAL SOCIETY, Band 62, Nr. 6, 8. Juni 1940, Seiten 1548-1549 C.D. HURD et al.: Acetoacetyl chloride Seiten 1548-1549**

(73) Patentinhaber: **LONZA AG, Gampel/Wallis (CH)**

(72) Erfinder: **Tenud, Leander, Dr., Balfrinstrasse 23, Visp (Kanton Wallis) (CH)**
Erfinder: **Miller, Raimund, Dr., Berkshire Place 66, Hackensack, N.J. 07601 (US)**
Erfinder: **Jackson, Barry Dr., Rathausstrasse 12, Visp (Kanton Wallis) (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 061 657 B1

## Beschreibung

Die vorliegende Erfindung betrifft ein Verfahren zur Herstellung von 2-Chloracetessigsäureamiden. 2-Chloracetessigsäureamide sind wichtige Zwischenprodukte zur Herstellung von Pestiziden und Herbiziden. Von 2-Chloracetessigsäureanilid leitet sich z.B. das Fungizid Vitavax® der Uniroyal Inc. ab.

Bei den bisher bekannten Herstellungsverfahren wird von den entsprechenden substituierten Acetessigsäureamiden, wie Acetessiganilid, augegangen, die entweder mit Chlorgas oder mit Sulfurylchlorid in Lösung oder Suspension chloriert werden. Die Chlorierung von Acetessigsäureamiden ist bekanntlich nur von geringer Selektivität. So wird die Rohausbeute an 2-Chloracetessigsäureanilid aus Acetessigsäureanilid mit 76% und einem Schmelzbereich von 122 bis 133°C (Smp Lit.: 138°C) angegeben (US-PS 3 852 351).

Die Chlorierung mit Sulfurylchlorid ist selektiver und liefert ein reines Endprodukt mit einem Smp von 136 bis 138°C (US-PS 3 249 499 und US-PS 3 393 202). Allerdings müssen die bei der Reaktion entstehenden Gase, $SO_2$ und HCl, getrennt und das $SO_2$ mit Chlor wieder in das Sulfurylchlorid übergeführt werden.

Nach der EP-A-0 005 849 können 2-Chloracetessigsäureamide durch Umsetzen von Acetessigsäureamiden mit stöchiometrischen Mengen von Chlor in Wasser hergestellt werden.

Ziel der vorliegenden Erfindung ist ein einfaches, leistungsfähiges Verfahren zur Herstellung von 2-Chloracetessigsäureamiden.

Erfindungsgemäss wird dies dadurch erreicht, dass man von Diketen ausgeht, dieses bei Temperaturen von +30 bis −40°C mit HCl in das Acetessigsäurechlorid überführt und durch Einleiten von Chlor bei Temperaturen von +30 bis −40°C das 2-Chloracetessigsäurechlorid bildet. Letzteres wird durch Zugabe von 1 Mol des entsprechenden Amines der allgemeinen Formel HNRR′ worin R und R′ gleich oder ungleich sind und H, Alkyl, substituiertes Alkyl, Aryl, substituiertes Aryl, Alkylaryl, substituiertes Alkylaryl, Alkoxyaryl, substituiertes Alkoxyaryl, Alkoxyalkyl, substituiertes Alkoxyalkyl bedeuten, und 1 Mol tertiärer Base, wie Triäthylamin, oder von 2 Molen des entsprechenden Amines bei Temperaturen von +50 bis−40°C in das entsprechende 2-Chloracetessigsäureamid übergeführt.

Die Chlorierung kann in Gegenwart von sauren Katalysatoren, wie Lewis-Säuren, z.B. $PCl_3$, $FeCl_3$, anorganischen Säuren, z.B. Schwefelsäure, Perchlorsäure, oder starken organischen Carbonsäuren, z.B. Essigsäure, Trifluoressigsäure, p-Toluolsulfonsäure, durchgeführt werden. Diese Säuren werden in katalytischen Mengen, zweckmässig 0,1 bis 20 Mol%, bezogen auf eingesetztes Diketen, zugesetzt.

Die Reaktionen können ohne Lösungsmittel, zweckmässig aber in einem Lösungsmittel, durchgeführt werden.

Als Lösungsmittel werden zweckmässig aromatische Kohlenwasserstoffe, wie Benzol, Toluol, oder chlorierte aliphatische Kohlenwasserstoffe, wie Tetrachlorkohlenstoff, Äthylchlorid, Dichloräthan usw., vorzugsweise Methylenchlorid, verwendet. Es können auch Lösungsmittelgemische eingesetzt werden.

Zweckmässig setzt man pro Mol Diketen 200 bis 1000 g Lösungsmittel ein.

Das Molverhältnis HCl zu Diketen in der ersten Stufe kann stark variieren, zweckmässig wird pro Mol Diketen 1,1 bis 10 Mol, vorzugsweise 1,95 bis 6,5 Mol HCl, angewendet.

Für die Chlorierung mit Chlor in der zweiten Stufe werden vorzugsweise pro Mol Acetessigsäurechlorid 0,9 bis 1,1 Mol Chlor angewendet.

Zur Herstellung der gewünschten Säureamide wird das in der zweiten Stufe erhaltene 2-Chloracetessigsäurechlorid mit dem entsprechenden Amin der allgemeinen Formel HNRR′ umgesetzt. Die Verbindung HNRR′ kann Ammoniak, ein primäres oder ein sekundäres Amin sein.

Nach speziellen Ausführungsformen werden als N-Verbindungen solche eingesetzt, welche eine gerade- oder verzweigtkettige Alkylgruppe mit 1 bis 6, insbesondere 1 bis 4, Kohlenstoffatomen aufweist, wobei auch entsprechende äthylenisch ungesättigte Gruppen, wie beispielsweise die Allylgruppe, anwesend sein können. Als Aryl- oder substituierte Arylgruppe liegt insbesondere die Phenyl- oder Benzylgruppe vor. Der Alkoxyrest in den genannten Gruppen ist vorzugsweise die Methoxygruppe. Als Substituenten der genannten Gruppen wird Chlor bevorzugt.

Als primäre Amine können Methyl-, Äthyl-, Propyl-, Isopropyl-, Methoxypropylamin, Anilin, 4-Cl-anilin, 4-Methoxyanilin, Benzylamin, Phenyläthylamin usw., als sekundäre Amine Dimethyl-, Diäthyl-, Methyläthyl-, Dipropyl-, Butyläthyl-, sek. Butyläthyl-, Methylisopropyl-, Allylmethyl-, Diäthylamin, N-Benzylanilin, N-Benzyl-p-(o,m)-toluidin usw. verwendet werden.

Eine besonders bevorzugte Ausführungsform der Erfindung besteht darin, dass man die drei Stufen im selben Reaktionsmedium und Reaktionsgefäss, ohne dass eines der Zwischenprodukte isoliert werden muss, durchführen kann.

Beispiel 1

Herstellung von 2-Chloracetessigsäureanilid.

In einem Doppelmantelkolben von 1000 ml Grösse, ausgestattet mit Pressluftrührer, Thermometer, Gaseinleitungsrohr mit Fritte, Rückflusskühler und Kryomat, wurden 50,4 g Diketen, gelöst in Methylenchlorid (300 ml) vorgelegt und auf −30°C abgekühlt. Nach Zugabe von 0,07 g (3 Tropfen) konzentrierter Schwefelsäure wurden in diese Lösung während 65 Minuten bei −30 bis −20°C 48,5 g Chlorwasserstoff eingeleitet. Nach einer Nachreaktionszeit von mindestens 1 Stunde wurden während 40 Minuten bei −25 bis −22°C 43,0 g Chlor eingeleitet und nach ½ Stunde der gebildete Chlorwasserstoff bei 20 Torr abgesaugt.

Zu dieser Lösung wurden nun während 46 Minuten bei −20 bis −8°C 110,8 g Anilin, gelöst in 130,0 Methylenchlorid, zugetropft. Die resultierende, schwer rührbare Suspension wurde auf Raumtemperatur aufwärmen gelassen und dann mit 200,0 g Wasser versetzt. Vom resultierenden Zweiphasengemisch wurde am Rotavapor die Hälfte des Methylenchlorids abdestilliert.

Das resultierende weisse Festprodukt wurde abgenutscht, 3 mal mit 30 ml Portionen Methylenchlorid gewaschen und bei 60°C im Vakuumtrokkenschrank während 4 Stunden getrocknet.

Ausbeute: 100,3 g (= 79,7% bezogen auf Diketen).

Die beiden Phasen des zurückgebliebenen Filtrates wurden getrennt und die Methylenchloridphase am Rotavapor zur Trockene eingedampft. Man erhielt auf diese Weise 26,6 g eines braunen Produktes, aus welchem durch Umkristallisation aus Äthanol 6,0 g Produkt (= 4,7% bezogen auf Diketen) gewonnen wurden. Gesamtausbeute: 106,3 g (= 84,4% bezogen auf Diketen) 2-Chloracetessigsäureanilid.

Beispiel 2

Herstellung von 2-Chloracetessigsäure-4'-chloranilid.

Zu einer Lösung von, analog Beispiel 1 aus 0,3 Mol Diketen bereitetem, 2-Chloracetessigsäurechlorid in 200 ml Methylenchlorid wurden während 15 Minuten bei einer Temperatur zwischen −30 bis −10°C 76,6 g 4-Chloranilin (6 Mol), gelöst in 200 ml Methylenchlorid, zugetropft. Nach 2 Stunden, nachdem sich die Temperatur auf 10°C eingestellt hatte, wurden 200 ml Wasser zugegeben und die resultierende Methylenchloridphase am Rotavapor auf zwei Drittel des Volumens eingeengt. Das anfallende leicht gräuliche Produkt wurde abfiltriert und getrocknet und ergab eine Rohausbeute von 63,3 g (= 84,4% bezogen auf Diketen).

Beispiel 3

Herstellung von 2-Chloracetessigsäure-4'-methoxyanilid.

Zu einer Lösung von, analog Beispiel 1 aus 0,3 Mol Diketen bereitetem, 2-Chloracetessigsäurechlorid in 200 ml Methylenchlorid wurden während 15 Minuten bei einer Temperatur zwischen −30 und −10°C 73,9 g 4-Methoxyanilin (0,6 Mol), gelöst in 200 ml Methylenchlorid, zugetropft und wie in Beispiel 2 beschrieben weitergearbeitet.

Als Ausbeute wurden 58,5 g eines leicht bräunlichen Produktes (= 80,7% Ausbeute, bezogen auf Diketen) erhalten.

Beispiel 4

Herstellung von 2-Chloracetessigsäurebenzylamid.

Analog den Beispielen 2 und 3 wurden 64,3 g Benzylamin (0,6 Mol) umgesetzt und 65,5 g eines weissen Produktes (= 96,7% Ausbeute, bezogen auf Diketen) erhalten.

Beispiel 5

Herstellung von 2-Chloracetessigsäure-2'-phenyläthylamid.

Analog den Beispielen 2 und 3 wurden 72,7 g 2-Phenyläthylamin (0,6 Mol) umgesetzt und 63,0 g leicht gelbliche Kristalle erhalten (= 87,6% Ausbeute, bezogen auf Diketen).

Beispiel 6

Herstellung von 2-Chloracetessigsäureisopropylamid.

Analog den Beispielen 2 und 3 wurden 35,5 g Isopropylamin (0,6 Mol) umgesetzt und 47,3 g leicht bräunliche Kristalle erhalten (= 88,8% Ausbeute, bezogen auf Diketen).

Beispiel 7

Herstellung von 2-Chloracetessigsäure-tert.butylamid.

Analog den Beisielen 2 und 3 wurden 43,9 g tertiäres Butylamin (0,6 Mol) umgesetzt und 51,3 g leicht bräunliche Kristalle erhalten (= 89,2% Ausbeute, bezogen auf Diketen).

Beispiel 8

Herstellung von 2-Chloracetessigsäure-3'-methoxypropylamid.

Analog den Beispielen 2 und 3 wurden 53,5 g 3-Methoxypropylamin (0,6 Mol) umgesetzt und 57,8 g einer leicht bräunlichen leicht beweglichen Flüssigkeit, die durch Destillation gereinigt wurde, erhalten (= 92,8% Ausbeute, bezogen auf Diketen).

Beispiel 9

Herstellung von 2-Chloracetessigsäuredimethylamid.

Analog den Beispielen 2 und 3 wurden 27,05 g Dimethylamin (0,6 Mol) umgesetzt und 41,2 g einer leicht beweglichen, durch Destillation gereinigten Flüssigkeit erhalten (= 83,9% Ausbeute, bezogen auf Diketen).

Beispiel 10

Herstellung von 2-Chloracetessigsäuremonomethylamid.

Analog den Beispielen 2 und 3 wurde aus 0,6 Mol Diketen 2-Chloracetessigsäurechlorid in 400 ml Methylenchlorid hergestellt und mit 37,3 g Methylamin (1,2 Mol) umgesetzt und 75,1 g eines durch Umkristallisation aus Wasser gereinigten Produktes erhalten (= 83,6% Ausbeute, bezogen auf Diketen).

Beispiel 11

Herstellung von 2-Chloracetessigsäureamid.

Analog den Beispielen 2 und 3 wurden aus 0,6 Mol Diketen 2-Chloracetessigsäurechlorid hergestellt und mit 20,5 g Ammoniak (1,2 Mol) umgesetzt und so 49,1 g eines durch Umkristallisation aus Diisopropyläther gereinigten Produktes erhalten (= 60,4% Ausbeute, bezogen auf Diketen).

Beispiel 12

Herstellung von 2-Chloracetessigsäureanilid.

Analog dem Beispiel 1 wurden 50,4 g Diketen in 300 ml Methylenchlorid gelöst und unter Rühren

auf 22°C aufgeheizt. Anschliessend wurden 0,14 g $H_2SO_4$ zupipettiert und dann bei 30°C innert 60 Minuten 48 g HCl-Gas durch die Lösung geleitet. Nach 30 Minuten wurden dann 43 g Chlorgas eingeleitet während 40 Minuten bei einer Temperatur von 30°C. Anschliessend wurde die Lösung bei 30°C mit 111 g Anilin während 20 Minuten versetzt und die Suspension für weitere 2 Stunden gerührt. Dann wurde die Suspension auf 10°C abgekühlt und mit Wasser 2 Stunden gerührt und dann filtriert, der Filterkuchen mit 10 ml Methylenchlorid gewaschen und dann getrocknet.

Man erhielt 49,4 g eines weissen Produktes, Smp 136 bis 137°C, entsprechend einer Ausbeute von 39,2%.

Die organische Phase vom Filtrat wurde eingeengt und weitere 59,6 g des Rohproduktes (das entsprach einer Ausbeute von 21%, mit einer Reinheit von 44,5%) erhalten.

Beispiel 13

Herstellung von 2-Chloracetessigsäureanilid.

Analog Beispiel 12 wurden die HCl-Anlagerung, die Chlorierung und die Amidierung mit Anilin bei 10°C durchgeführt. Nach Aufarbeitung in derselben Weise wurden 58,0 g Produkt, Smp 136 bis 138°C, entsprechend einer Ausbeute von 46,0%, erhalten.

Nach Einengen der organischen Phase des Filtrats wurden weitere 64,2 g des Rohproduktes (das entspricht einer Ausbeute von 21,9%, mit einer Reinheit von 43,7%) erhalten.

Beispiel 14

Herstellung von 2-Chloracetessigsäureanilid.

In einer Apparatur wie in Beispiel 1 wurden 50,4 g Diketen, gelöst in 100 ml Methylenchlorid, vorgelegt und auf −10°C abgekühlt. Während 60 Minuten wurden 48 g Chlorwasserstoff bei −10°C eingeleitet und anschliessend während 30 Minuten gerührt. Dann wurde die Reaktionsmischung auf −20°C gekühlt und bei dieser Temperatur 41 g Chlorgas während 1 Stunde eingeleitet. Die Lösung wurde anschliessend bis 10°C aufgeheizt und der entstandene Chlorwasserstoff unter Vakuum abgesaugt. Zu dieser Lösung wurden 400 ml Toluol zugegeben und dann 111 g Anilin während 40 Minuten zugetropft, dann wurde die Suspension während weiterer 2 Stunden gerührt. 200 ml Wasser wurden zugegeben und die Suspension während 15 Minuten gerührt, dann filtriert und der Kuchen mit 100 ml Wasser und 60 ml Toluol gewaschen und anschliessend getrocknet.

Man erhielt 102,7 g Produkt, Smp 134 bis 135°C, entsprechend einer Gewichtsausbeute von 81,4%.

Die organische Phase des Filtrats lieferte nach Einengen einen klebrigen Rückstand (20,6 g).

## Patentansprüche

1. Verfahren zur Herstellung von 2-Chloracetessigsäureamiden, dadurch gekennzeichnet, dass man Diketen bei Temperaturen von +30 bis −40°C mit Hilfe von Chlorwasserstoff in das Acetessigsäurechlorid überführt, durch Einleiten von Chlor bei Temperaturen von +30 bis −40°C das 2-Chloracetessigsäurechlorid bildet und letzteres durch Umsetzung mit N-Verbindungen der allgemeinen Formel

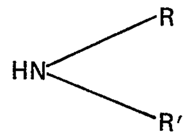

wobei R = R′ = H
oder R = R′ = Alkyl, substituiertes Alkyl, Aryl, substituiertes Aryl, Alkylaryl, substituiertes Alkylaryl, Alkoxyaryl, substituiertes Alkoxyaryl, Alkoxyalkyl, substituiertes Alkoxyalkyl,
oder R = H, R′ = Alkyl, substituiertes Alkyl, Aryl, substituiertes Aryl, Alkylaryl, substituiertes Alkylaryl, Alkoxyaryl, substituiertes Alkoxyaryl, Alkoxyalkyl, substituiertes Alkoxyalkyl

bedeuten kann, bei Temperaturen von +50 bis −40°C in das entsprechende Amid überführt.

2. Verfahren nach Patentanspruch 2, dadurch gekennzeichnet, dass man N-Verbindungen mit Alkylen, Alkoxyalkylen bzw. Alkoxyarylen mit 1 bis 20 C-Atomen verwendet.

3. Verfahren nach Patentansprüchen 1 und 2, dadurch gekennzeichnet, dass man die Chlorierung in Gegenwart von sauren Katalysatoren durchführt.

4. Verfahren nach Patentansprüchen 1 bis 3, dadurch gekennzeichnet, dass man die Chlorierung in einem organischen Lösungsmittel durchführt.

5. Verfahren nach Patentanspruch 1, dadurch gekennzeichnet, dass man die Dreistufenreaktion ohne Isolierung der Zwischenprodukte durchführt.

## Claims

1. Process for the preparation of 2-chloro aceto acetic acid amides, characterized in that diketene is converted into aceto acetic acid chloride at temperatures of +30 to −40°C by means of hydrogen chloride, by introducing chlorine at temperatures of +30 to −40°C the 2-chloro aceto acetic acid chloride is formed, and the latter is converted into the corresponding amide by reacting it with N-compounds of the general formula

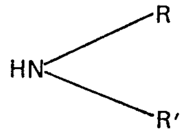

wherein R = R′ = H
or R = R′ = alkyl, substituted alkyl,
aryl, substituted aryl,
alkylaryl, substituted alkylaryl,
alkoxyaryl, substituted alkoxyaryl,
alkoxyalkyl, substituted alkoxyalkyl
or R = H,
R′ = alkyl, substituted alkyl,
aryl, substituted aryl,
alkylaryl, substituted alkylaryl,
alkoxyaryl, substituted alkoxyaryl,
alkoxyalkyl, substituted alkoxyalkyl
at temperatures of +50 to −40°C.

2. Process according to claim 1, characterized in that N-compounds with alkylenes alkoxyalkylenes and alkoxyarylenes respectively, of 1 to 20 C-atoms are used.

3. Process according to claims 1 and 2, characterized in that the chlorination is carried out in the presence of acidic catalysts.

4. Process according to claims 1 to 3, characterized in that the chlorination is carried out in an organic solvent.

5. Process according to claim 1, characterized in that the three-step-reaction is carried out without isolation of intermediate products.

## Revendications

1. Procédé de préparation de 2-chloroacétoacétamides caractérisé en ce qu'on transforme le dicétène à des températures de +30 à −40°C, à l'aide de chlorure d'hydrogène, en le chlorure d'acétoacétyle, on forme, en faisant passer du chlore à des températures de +30 à −40°C, le chlorure de 2-chloroacétoacétyle, et on transforme ce dernier en l'amide correspondant par réaction avec des N-composés répondant à la formule générale:

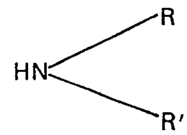

dans laquelle R = R′ = H
ou R = R′ = alcoyle, alcoyle substitué,
aryle, aryle substitué,
alcoylaryle, alcoylaryle substitué,
alcoxyaryle, alcoxyaryle substitué,
alcoxyalcoyle, alcoxyalcoyle substitué,
ou R = H,
R′ = alcoyle, alcoyle substitué,
aryle, aryle substitué,
alcoylaryle, alcoylaryle substitué,
alcoxyaryle, alcoxyaryle substitué,
alcoxyalcoyle, alcoxyalcoyle substitué,
à des températures de +50 à −40°C.

2. Procédé suivant la revendication 1, caractérisé en ce qu'on utilise des N-composés avec alcoylène, alcoxyalcoylène ou alcoxyarylène avec 1 à 20 atomes de C.

3. Procédé suivant les revendications 1 et 2, caractérisé en ce qu'on effectue la chloration en présence de catalyseurs acides.

4. Procédé suivant les revendications 1 à 3, caractérisé en ce qu'on effectue la chloration dans un solvant organique.

5. Procédé suivant la revendication 1, caractérisé en ce qu'on effectue la réaction en 3 stades sans isolement des produits intermédiaires.